# EUROPEAN PATENT APPLICATION

(11) **EP 3 977 931 A1**
(43) Date of publication of application: **06.04.2022**
(21) Application number: 20200013.9
(22) Date of filing: 05.10.2020
(51) Int. Cl.: A61B 5/22, A61B 5/00, A61B 5/11

(54) **HIP ABDUCTOR MUSCLE STRENGTH MEASUREMENT SYSTEM**

(71) Applicant: Haute École Spécialisée de Suisse Occidentale - Genève (HES-SO Genève), 1227 Carouge (CH); Les Hôpitaux Universitaires de Genève, 1205 Geneva (CH); UNIVERSITE DE GENEVE, 1211 Geneva 4 (CH)
(72) Inventor: ALLET, Lara, 1981 VEX (CH); GAFNER, Simone, 1205 Genève (CH); PASSERAUB, Philippe, 1680 Romont (CH); PRAZ, Quentin, 01280 Prévessin-Moëns (FR)
(74) Representative: reuteler & cie SA

(57) **Abstract**

A hip abductor muscle force measurement system (2) comprises a force measurement device (6) and a waist belt (4) for securing the force measurement device around the waist of a patient. The force measurement device comprises a housing (10), an electronic circuit (20) installed inside the housing, a force measurement contact pad (14) and a force sensor (16) coupled between the force measurement contact pad and the housing arranged for measuring a compression force on the contact pad biased towards the housing, the system operable to measure at least a maximum force and a rate of force generation.

## Description

The present invention relates to a system for assessing hip abductor muscle strength.

Hip abductor muscle strength is closely related to a person's risk of falling, in particular for elderly persons. In physical therapy performed in hospital or outpatient practices, physical therapists carry out functional performance assessments of patients to determine the underlying fall risk factor and to better target rehabilitation of the patient. More generally, there is an increasing interest in assessing elderly persons hip abductor muscle strength. It has been found that elderly people especially those persons that have a high risk of falling, have a reduced mediolateral stability while standing and an increased mediolateral body motion during dynamic balance recovery and forward stepping tasks. This mediolateral control is mainly assured through proximal hip frontal plane muscle strength. Recent studies showed that hip abductor muscle strength is an important hip muscle group to distinguish between older adult fallers and non-fallers. Hip abductor strength should thus be considered when assessing older persons fall risk. Currently used hip abductor strength tests are either too time consuming or not precise enough to be used as a clinical measure. Moreover, existing tests may require specialized equipment and/or require the patient to be in uncomfortable positions during the test, for instance lying down.

Moreover, in addition to hip abductors maximum voluntary isometric muscle strength, an important parameter in determining a patient's capacity to recover from an unstable position instead of falling is the patient's rate of development of muscle force.

In view of the foregoing, it is an object of this invention to provide a system for measuring hip abductor muscle strength that is easy and convenient to use, and that provides reliable, accurate and pertinent information.

It is advantageous to provide a system for measuring hip abductor muscle strength that allows assessing a person's risk of falling and ability to recover from an unstable position.

It is advantageous to provide a system for measuring hip abductor muscle strength that is economical to produce and to use.

It is advantageous to provide a hip abductor muscle strength measurement device that is light and compact.

It is advantageous to provide a hip abductor muscle strength measurement device that can be easily used outside of a hospital setting and without highly qualified health care personnel.

It is advantageous to provide a hip abductor muscle strength measurement device that may be used in non-medical applications, for instance for sports training.

Objects of the invention have been achieved by providing a hip abductor muscle strength measurement system according to claim 1, and a method of assessing hip abductor muscle strength according to claim 12. Dependent claims recite various advantageous features of the invention.

Disclosed herein is a hip abductor muscle force measurement system comprising a force measurement device and a waist belt for securing the force measurement device around the waist of a patient. The force measurement device comprises a housing, an electronic circuit installed inside the housing, a force measurement contact pad and a force sensor coupled between the force measurement contact pad and the housing arranged for measuring a compression force on the contact pad biased towards the housing, the system operable to measure at least a maximum force and a rate of force generation.

In an advantageous embodiment, the force measurement contact pad comprises a convex rounded contact surface.

In an advantageous embodiment, the contact surface comprises a non-slip material such as rubber.

In an advantageous embodiment, a patient side of the housing has a concave curved shape to conform to a patient's body shape.

In an advantageous embodiment, the force measurement contact pad is movably mounted with respect to the housing and may comprise guides to position and guide the force contact pad in a direction of translation parallel to the application of pressure against the contact pad towards the housing.

In an advantageous embodiment, the guides comprise guide pins arranged at corners of the force measurement contact pad.

In an advantageous embodiment, the force measurement device further comprises a user interface including a screen operable for displaying force measurement results.

In an advantageous embodiment, the electronic circuit comprises at least a microprocessor, a memory, an analog to digital converter for converting an analog signal from the force measurement sensor to a digital signal, a communication module for wireless communication with an external computing device, and an autonomous power source.

In an advantageous embodiment, the electronic circuit further comprises a digital filter for smoothing the signal.

In an advantageous embodiment, the force measurement device is operable to measure a maximum force applied and a rate of force generation over a predefined time span

In an advantageous embodiment, the force measurement device is operable to measure a rate of force generation over an initial time span ***ti*** within 0.5 to 6 seconds after measurement start, preferably within 0.5 to 2 seconds.

In an advantageous embodiment, the force measurement device comprises a force set button to start the force measurement test.

Also disclosed herein is a method of assessing hip abductor muscle strength using a hip abductor muscle force measurement system, comprising the steps of conducting a hip abductor muscle strength measurement test during a time period in a range having an amplitude of 3 to 10 seconds, converting in the electronic system an analog output of the force sensor to a digital signal, detecting a maximum force value within said time period, said maximum force corresponding to a maximum voluntary isometric strength (MVIS), and computing a rate of force generation (RFG) within an initial predefined time span.

In an advantageous embodiment, the initial predefined time span for the RFG is mostly in a time-window of an amplitude between 20ms to 100ms, preferably between 40ms to 80ms, said time window starting after about 5-15%, preferably around 10%, of the detected maximum force.

In an advantageous embodiment, measurement results are transmitted wirelessly from the force measurement device to an external computing device.

In an advantageous embodiment, said MVIS and RFG values are generated by the electronic circuit within the force measurement device.

Further objects and advantageous features of the invention will be apparent from the claims, from the detailed description, and annexed drawings, in which:
Figure 1a is a perspective view of a hip abductor muscle strength measurement device, shown without belt, according to an embodiment of the invention;
Figure 1b is a view of front face of the device of figure 1a;
Figure 1c is an exploded perspective view of the device of figure 1a;

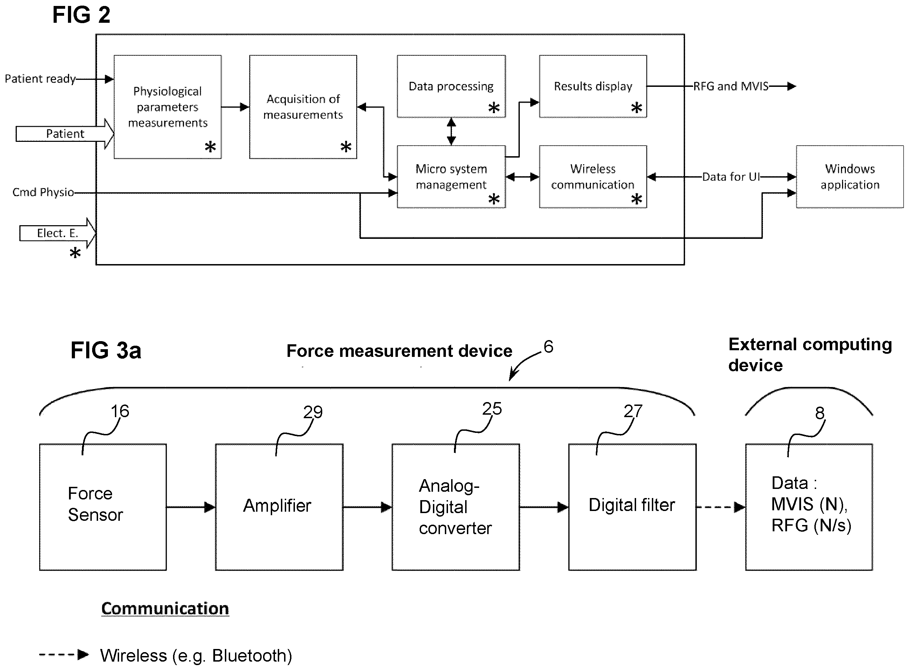

Figure 2 is a schematic block diagram of a hip abductor muscle strength measurement system according to an embodiment of the invention;

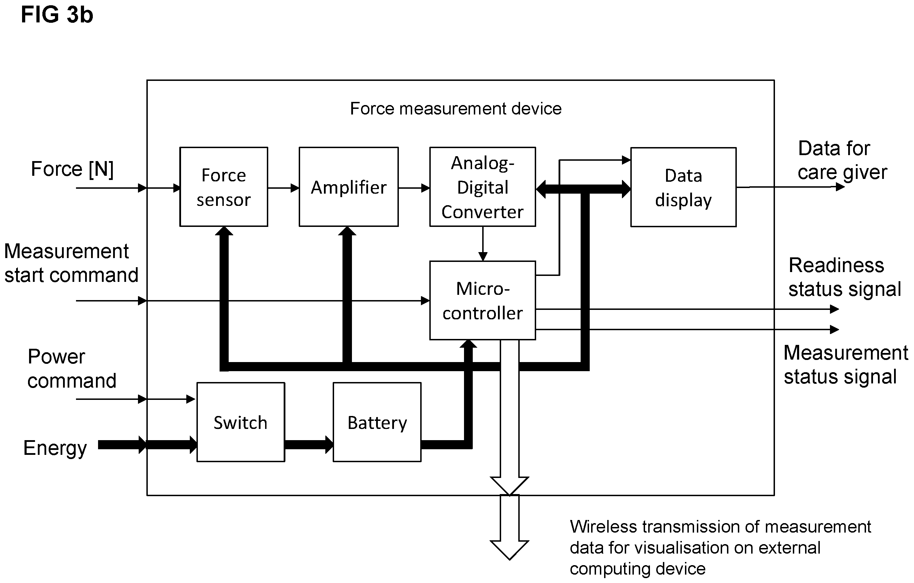

Figures 3a, 3b are schematic simplified block diagrams of functional components of a hip abductor muscle strength measurement system according to an embodiment of the invention;

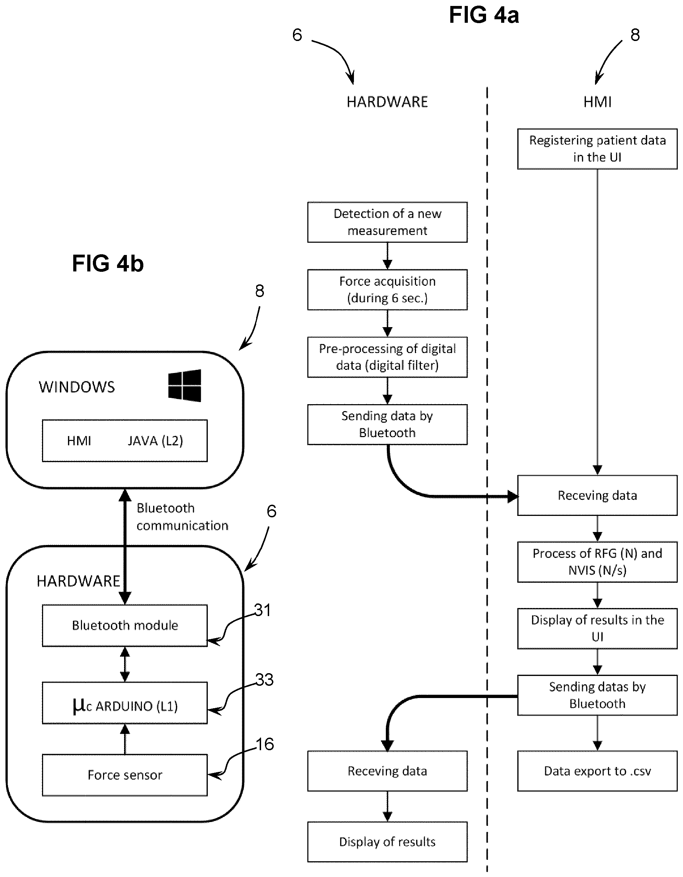

Figure 4a is a schematic flow diagram of a measurement process using a hip abductor muscle strength measurement device according to an embodiment of the invention;
Figure 4b is a simplified schematic block diagram showing a communication of data between a hip abductor muscle strength measurement device and an external computing system according to an embodiment of the invention;

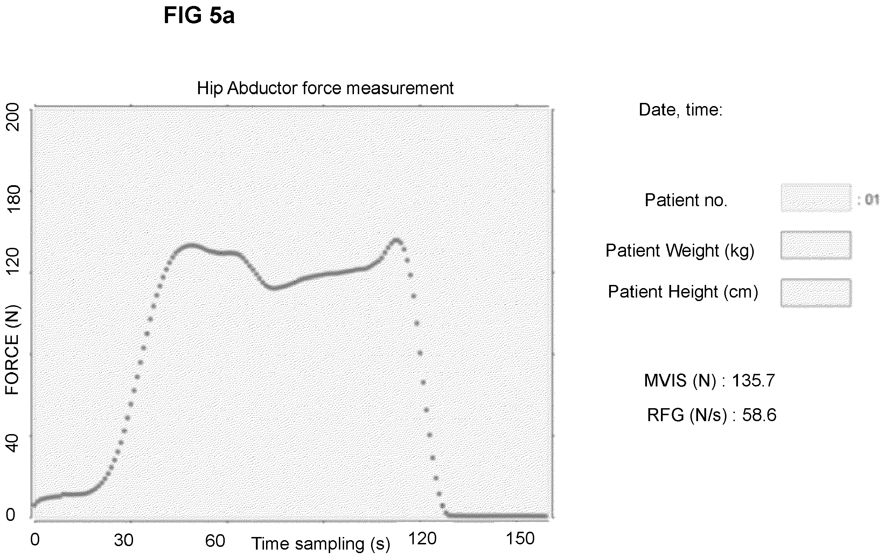

Figure 5a is a plot of measured force over time of a patient's hip abductor muscle strength measured using a hip abductor muscle strength measurement according to an embodiment of the invention;

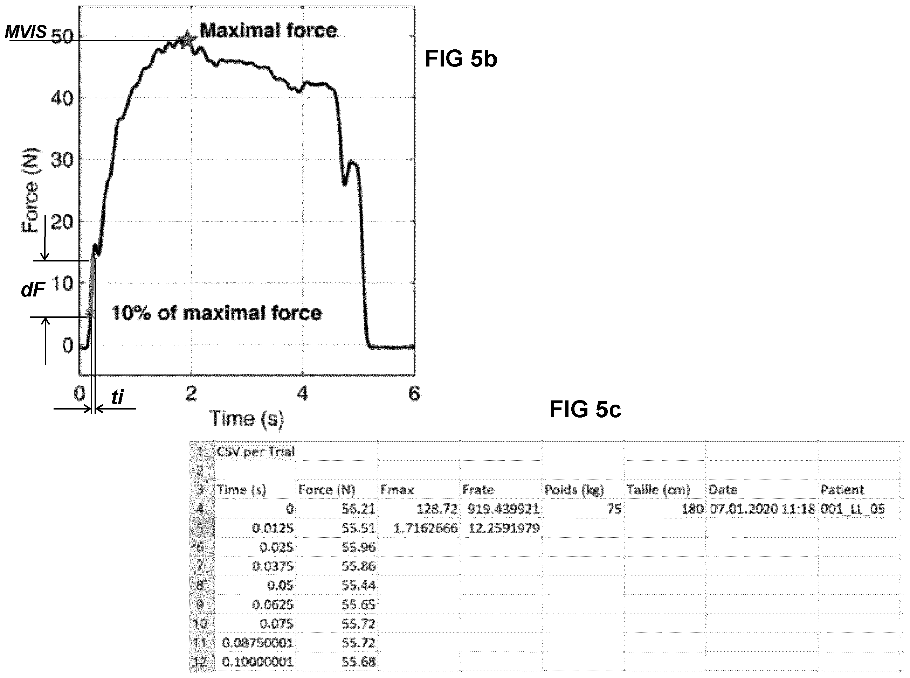

Figure 5b is another plot similar to figure 5a;
Figure 5c is an example of data (force over time) output by a hip abductor muscle strength measurement device according to an embodiment of the invention;

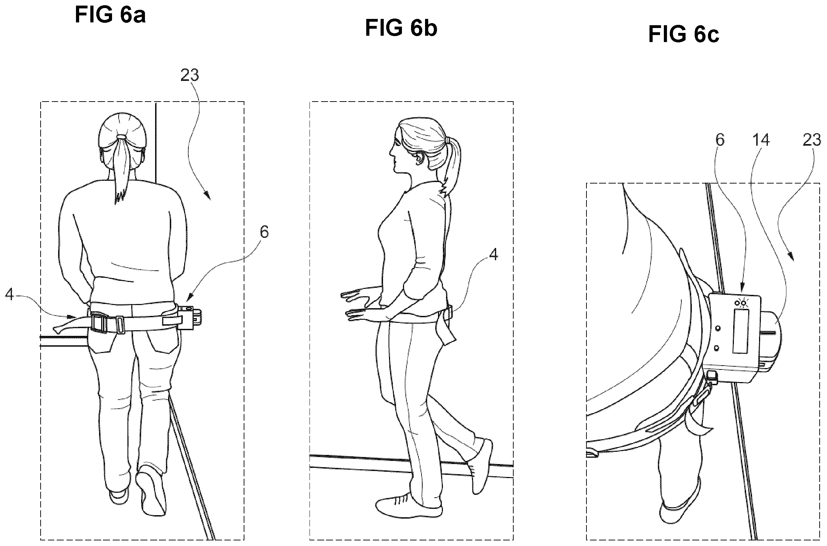

Figures 6a to 6c are different views of a patient undergoing a hip abductor muscle strength assessment using a system according to an embodiment of the invention.

Referring to the figures, a hip abductor muscle force measurement system 2 comprises a force measurement device 6, a waist belt 4 for securing the force measurement device around the waist of a patient, and an external computing device 8 configured to receive measurement output data wirelessly from the force measurement device 6.

The force measurement device 6 comprises a housing 10 having thereon belt anchors 22 for securing the waist belt 4 to the housing, a force measurement contact pad 14, and a force sensor 16 coupled between the force measurement contact pad 14 and the housing 10. The force measurement device 6 further comprises an electronic circuit 20 installed inside the housing and a user interface 12 with inputs, such as buttons 12b-12c for switching on/off and entering commands (e.g. for commencing measurement), and outputs, such as a screen 12a for displaying information or LED status indicators 12d to indicate for instance ready for measurement (e.g. green) or measurement in progress (e.g. red).

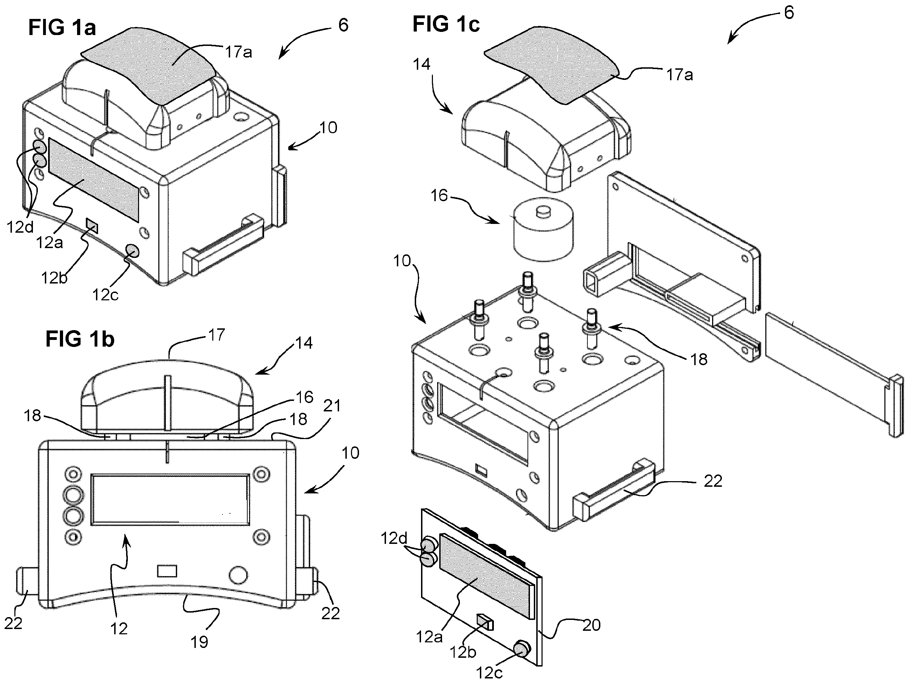

The force measurement contact pad comprises a contact surface 17 that preferably comprises a convex curved surface, for instance as best illustrated in figures 1a-1c, configured for application against a substantially planar surface, in particular of a wall 23 while the patient is standing. The convex curved contact surface 17 may be configured to have a substantially line band contact with the substantially planar surface against which the contact surface is pressed. The contact surface 17 preferably comprises a non-slip compliant material 17a that has a high frictional force with surfaces such as walls and other upstanding surfaces against which a patient is expected to lean against. The frictional force of contact between the non-slip compliant material and wall provides stability for the patient while he/she is leaning in a standing position against the wall. The non-slip compliant material 17a may advantageously comprise or consist of an elastomeric layer such as a rubber layer. The force measurement contact pad 14 may thus be coated with a rubber or similar elastomeric material forming the contact surface 17.

The housing 10 and force measurement contact pad 14 may be made of a plastics material such as ABS (Acrylnitril-Butadien-Styrol-Copolymer) or other polymers that are molded for a light and economical construction.

The force measurement contact pad 14 is movably mounted with respect to the housing 10 and may comprise guides 18 to position and guide the force contact pad in a direction of translation parallel to the application of pressure against the contact pad towards the housing 10. The guides may for instance be in the form of guide pins arranged at corners of the force measurement contact pad 14, as illustrated.

The force sensor 16 is mounted between the contact pad 14 and the housing 10 and is configured to measure compression force, in particular when the force measurement contact pad 14 is pushed towards the housing 10. As best seen in figures 1a-1c in conjunction with 6a-6c, a patient side 19 of the housing 10 is pressed against a patient's hip when the belt 4 is secured around the patient's waist, and the force measurement contact pad positioned on the opposite outer side 21 of the housing and applies a reactive force towards the patient as the patient pushes himself towards the upstanding surface 23. The compressive force is fully taken up by the force sensor 16 which couples the force measurement contact pad 14 to the housing 10. The patient side 19 of the housing preferably has a concave curved shape to conform somewhat to a patient's hip shape.

The force measurement device 6 is operable to measure not only the maximum force applied but also the development of the force over time. The rate of development of force for a selected period of measurement time may thus be determined, such measurements being useful in the assessment of the patient's hip abductor muscle strength.

The electronic circuit 20 installed within the housing 10 comprises at least a micro-processor 33, a memory, an analog to digital converter 25, a communication module 31 for wireless communication (e.g. using bluetooth and/or Wifi protocols) with an external computing device 8, and an autonomous power source such as a battery.

The force sensor 16 may be for instance comprise a sensor of a capacitive, inductive, resistive, magnetic or piezoelectric type, for instance a piezoresistive gauge based force sensor. The force sensor 16 emits an analog force measurement signal that is converted to a digital signal by an analog to digital converter 25 which may then be filtered by a digital filter 27 before being output to the external computing device 8. The digital filter serves to smooth the signal, for instance by means of a moving average type of filter, *per se* well known in the field of signal smoothing filters. Further processing of the digital signal may be performed by the electronic circuit 20 prior to being output to the external computing device.

The analog signal output by the force sensor may be amplified by an amplifier 29 of the electronic circuit 20 prior to conversion to a digital signal. The digital filter 27 may comprise a low pass or smoothing filter in order to eliminate noise and high frequency signals in order to obtain a smooth force over time measurement curve.

In a hip abductor muscle strength measurement test according to the invention, the force measurement may be taken over period typically in a range of 3 to 10 seconds, for instance around 5 to 7 seconds. When a patient is asked to press as hard as possible with his/her hip abductor muscle, the force measured over an initial time span ***ti***, for instance within 0.5 to 2 seconds after measurement start, provides a value of the rate of force generation (RFG) ***dF*/*dt*** that is a useful parameter indicative of a patient's capacity of recovery from an unstable position. In addition, the maximum force is captured from the force over time measurement curve. The stability of holding the maximum force over a few seconds may also be a useful parameter for assessing a patient's hip abductor muscle strength.

The user interface 12 may advantageously comprise a screen 12a that provides some information on the status of the device, for instance indicating when the device is on and providing instructions for initializing the device and commencing the measurement process. The user interface may also be used to display measurement data such as the maximum force and the rate of force generation directly on the device. Prior to commencing a measurement operation, the device must be turned on by switching the power button 12b. This action switches on the measurement systems and initializes it right before the measurement is started and the patient is applying muscle strength. Thus, if the patient is leaning more or less hard against the wall prior to starting the pushing action, the base force prior to application of the muscle force will be subtracted from the measurement result using the force reset button.

The device may also emit sounds in order to indicate starting and stopping of the measurement process or indicating alarms of faulty or improper measurement processes.

Force measurement data may be filtered and processed by the electronic circuit 20 in order to reduce the amount of data transferred externally, for instance by cropping the measurement time window and/or reducing the number of samples transferred, and/or to format the data, for instance by outputting force over time measurement results in a specific format. The aforementioned specific format may for instance include a .csv format or other standardized formats that may be easily converted into commonly used programs such as MS Word, Excel and the like, and to generate plots of the output results for visualization by the user or healthcare practitioner.

An example of a measurement step according to an embodiment of the invention is described hereafter. The following described example is illustrative of a specific example of implementation of the invention that can however vary in terms of instructions given to the patient, times for applying pressure and other variables.

### Example: hip abductor strength test measurement process

The measurement program may be started on the external computing device by entering the participants weight, height, and tested leg side (left or right leg). Fix the force measurement device 6 on the greater trochanter of the participant's hip close to the wall. Adjust the belt 4 so that it holds on the hip (great trochanter) of the participant without manual support. Position the participant in the measurement position, which is in an upright position close to the wall with the hip in a neutral position in the sagittal and frontal plane. The participant's shoulder is not touching the wall. Verify that the force measurement device is horizontal also when the participant raises his /her leg close to the wall. The contact pad 14 touches the wall surface 23. A chair or other support may be placed in front of the participant to allow the participant to assure a stable position (participants are allowed to touch the chair or support but not to push or pull on it). The non-tested leg (the leg close to the wall) is then raised up and the knee flexed in an approximately 20° position. The tester may stand behind the participant to guides him/her in the correct starting position by tactile and verbal information. One to three test trials may for instance be performed until the participant understands the test movement.

Participants' are then asked to push with the standing leg (the one further away from the wall) as hard and as fast as possible in the direction of the wall in order to push the pelvis with the attached force measurement device 6 against the wall. The command to initiate the test may for instance be as follows: "are you ready?", "go" and the participant will be verbally encouraged to "push", "push", "push" against the wall over a specified measurement time span, for instance around 5 seconds. The test may be repeated a number of times per leg, for instance three times per leg.

The participant may be asked about his/her dominant leg (leg they would use to kick a ball with). If the participant is not sure, this may be tested with an object put on the floor.

The data transferred from the force measurement device to the external computing device may be stored in a folder "DATA" on the external computing device for instance as csvfiles. DATA may also include raw strength data as well as maximum voluntary isometric strength (MVIS), normalized MVIS (normalized data by bodyweight) and rate of force generation (RFG measured in normalized bodyweight seconds).

When one uses the RESUME button at the end of the measuring day or when several tests are saved in "data" one gets a *csv* file "RESUME" in which the participants ID and strength values are summarized as non-normalized values.

Measurement results may also be displayed on the screen 12a of the force measurement device, optionally as a visual control of the measurement results transmitted to the external computing device 8. The display of results on the device screen 12a may also serve to check if something abnormal happened during the measurement process.

To measure maximum MVIS as well as the RFG, participants are asked to push the force measurement sensor with their hip abductors of the opposite side as hard and fast as possible against the wall, hold the pressure for about five seconds and relax. The MVIS is defined as the highest force generated by the subject within a specified time span from the beginning of the test, for instance a time span of up to about four seconds. The RFG is evaluated within an initial specified time span, for instance a 50-ms time-window after about 10% of the MVIS was reached. The measuring device may output the raw strength data as well as by body weight normalized data for MVIS and RFG. Participants may typically repeat the test three times per leg with a break of about 30 seconds between each trial. The mean value of the three individual trials per leg and the mean value of the six trials can be retained for further analyses. Probable leg asymmetries can thus be assessed.

### List of references used

Hip abductor muscle force measurement system 2
**waist belt 4**
**force measurement device 6**
   **housing 10**
      belt anchor 22
      patient side 19
      outer side 21
   **user interface 12**
      outputs (e.g. screen 12a, status lights 12d)
      inputs (e.g. buttons 12b (power), 12c (e.g. measurement start/stop))
   **force measurement contact pad 14**
      contact surface 17
      guides 18
         guide pins
   **force sensor 16**
   **electronic circuit 20**
      microprocessor 33
      memory
      A/D converter 25
      acquisition module
         amplifier 29
      data processing module
         digital filter 27
      communication module (wireless) 31
         e.g. bluetooth / wifi
      power source (battery)
**external computing device 8**
**wall surface 23**
initial time span ***ti***
rate of force generation (RFG) ***dF*/*dt***
maximum voluntary isometric strength (MVIS)

## Claims

1. A hip abductor muscle force measurement system (2) comprises a force measurement device (6) and a waist belt (4) for securing the force measurement device around the waist of a patient, the force measurement device comprising a housing (10), an electronic circuit (20) installed inside the housing, a force measurement contact pad (14) and a force sensor (16) coupled between the force measurement contact pad and the housing arranged for measuring a compression force on the contact pad biased towards the housing, the system operable to measure at least a maximum force and a rate of force generation.

2. The hip abductor muscle force measurement system according to the preceding claim, wherein the force measurement contact pad comprises a convex rounded contact surface (17).

3. The hip abductor muscle force measurement system according to the preceding claim, wherein the contact surface comprises a non-slip material such as rubber.

4. The hip abductor muscle force measurement system according to any preceding claim, wherein a patient side (19) of the housing has a concave curved shape to conform to a patient's hip shape.

5. The hip abductor muscle force measurement system according to any preceding claim, wherein the force measurement contact pad is movably mounted with respect to the housing and may comprises guides (18) to position and guide the force contact pad in a direction of translation parallel to the application of pressure against the contact pad towards the housing.

6. The hip abductor muscle force measurement system according to the preceding claim, wherein the guides comprise guide pins arranged at corners of the force measurement contact pad.

7. The hip abductor muscle force measurement system according to any preceding claim, wherein the force measurement device further comprises a user interface (12) including a screen (12a) operable for displaying force measurement results.

8. The hip abductor muscle force measurement system according to any preceding claim, wherein the electronic circuit comprises at least a microprocessor (33), a memory, an analog to digital converter (25) for converting an analog signal from the force measurement sensor to a digital signal, a communication module (31) for wireless communication with an external computing device, and an autonomous power source.

9. The hip abductor muscle force measurement system according to any preceding claim, wherein the force measurement device is operable to measure a maximum force applied and a rate of force generation over a predefined time span

10. The hip abductor muscle force measurement system according to any preceding claim, wherein the force measurement device is operable to measure a rate of force generation over an initial time span ***ti*** within 0.5 to 6 seconds after measurement start, preferably within 0.5 to 2 seconds after measurement start.

11. The hip abductor muscle force measurement system according to any preceding claim, wherein the force measurement device comprises a force set button to set the compression force at a predefined starting value prior to a force measurement test.

12. Method of assessing hip abductor muscle strength using a hip abductor muscle force measurement system according to any preceding claim, comprising the steps of conducting a hip abductor muscle strength measurement test during a time period in a range of 3 to 10 seconds, converting in the electronic system an analog output of the force sensor to a digital signal, detecting a maximum force value within said time period, said maximum force corresponding to a maximum voluntary isometric strength (MVIS), and computing a rate of force generation (RFG) within an initial predefined time span.

13. Method according to the preceding claim wherein the initial predefined time span is in a time-window of magnitude between 20ms to 100ms, preferably between 40ms to 80ms, said time window starting after about 5-15%, preferably around 10%, of the detected maximum force.

14. Method according to either of the two directly preceding claims wherein measurement results are transmitted wirelessly from the force measurement device to an external computing device.

15. Method according to any of the three directly preceding claims wherein said MVIS and RFG values are generated by the electronic circuit within the force measurement device.
